# EUROPÄISCHE PATENTANMELDUNG

(11) **EP 3 964 255 A1**
(43) Veröffentlichungstag der Anmeldung: **09.03.2022**
(21) Anmeldenummer: 21195362.5
(22) Anmeldetag: 07.09.2021
(51) Int. Cl.: A61M 39/10, F04B 43/12, F04B 43/00, F04B 13/00

(54) **DISPENSIER-KASSETTE**

(30) Priorität: 08.09.2020 CH 11122020
(71) Anmelder: INTEGRA Biosciences AG, 7205 Zizers (CH)
(72) Erfinder: Shearer, Daniel, 7415 Pratval (CH); Pasquier, Noel, 7302 Landquart (CH); Städler, Andreas, 7012 Felsberg (CH)
(74) Vertreter: Riederer Hasler & Partner Patentanwälte AG

(57) **Zusammenfassung**

Dargestellt und beschrieben ist eine Dispensier-Kassette zum Anbringen an einer Peristaltikpumpe mit einer ersten Kartusche, einer zweiten Kartusche und Schläuchen, welche von der Dispensier-Kassette derart aufgenommen werden, dass beide Enden der Schläuche je in einer Kartusche angebracht sind.

Erfindungsgemäss ist vorgesehen, dass die zwei Kartuschen über ein elastisches Mittelstück miteinander verbunden sind.

## Beschreibung

### TECHNISCHES GEBIET DER ERFINDUNG

Die Erfindung betrifft eine Dispensierkassette zur Verwendung in einer Peristaltikpumpe gemäss Oberbegriff des Anspruchs 1.

### HINTERGRUND DER ERFINDUNG

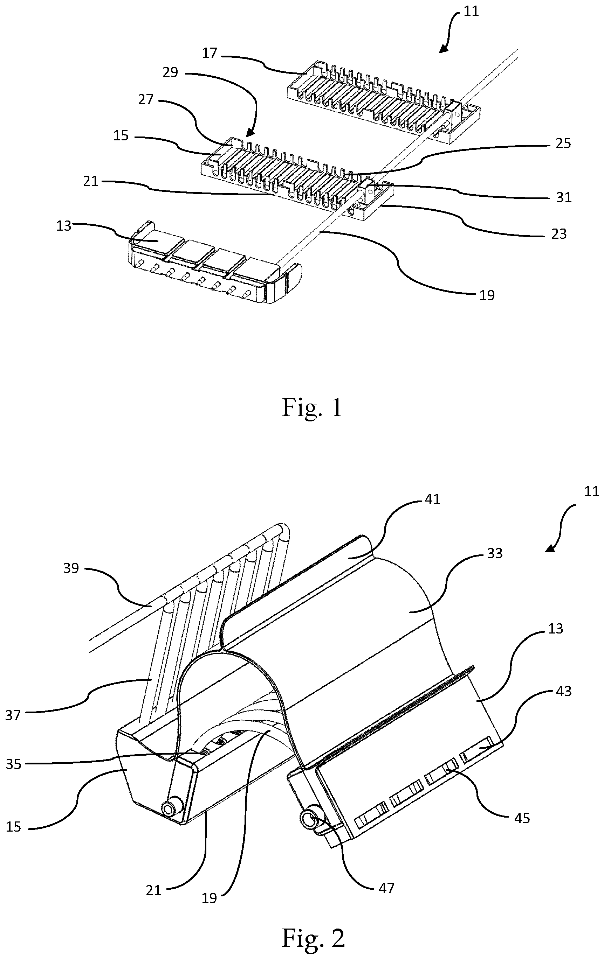

Die aus dem Stand der Technik bekannten Dispensierkassetten zur Anwendung in einer Peristaltikpumpe weisen einen dreiteiligen Aufbau auf. Die Dispensier-Kassette umfasst drei Kartuschen sowie durchgehende Schläuche, welche jeweils von der ersten Kartusche angefangen über die zweite Kartusche zur dritten Kartusche führen (siehe Figur 1). Die Schläuche erzeugen die einzige Verbindung zwischen den Kartuschen. Die Anzahl der Schläuche in einer Dispensierkassette, welche nebeneinander in einer Reihe und parallel zueinander angeordnet sind, beträgt 8 oder 16. Die Schläuche zwischen der ersten und zweiten Kartusche kommen in einer Peristaltikpumpe auf deren Rotor zu liegen. Aufgrund der Tatsache, dass die Schläuche zwischen der ersten und zweiten Kartusche auf dem Rotor angeordnet und dadurch grösseren Beanspruchungen ausgesetzt sind, können diese Schläuche separat zur Verfügung gestellt sein und brauchen nicht die ganze Länge von der ersten bis zur dritten Kartusche abzudecken. In der ersten Kartusche sind nebst den Schläuchen auch Dispensier-Spitzen angebracht, welche das in den Schläuchen transportierte Medium nach aussen fördern. Bei einer aus dem Stand der Technik bekannten Dispensier-Kassette muss jede Kartusche einzeln an der Peristaltikpumpe angebracht werden. Bei einem dreiteiligen Aufbau der Dispensier-Kassette führt dies zu drei nacheinander durchzuführenden Arbeitsschritten, welche sowohl beim Anbringen an als auch beim Lösen von der Peristaltikpumpe vorzunehmen sind.

Die in einer Dispensier-Kassette eingesetzten Schläuche müssen eine flexible Aussenwand aufweisen. Die Beförderung des Mediums in den Schläuchen geschieht durch die Verwendung der Peristaltik. Das heisst, dass aufgrund der Bewegung des Schlauches das Medium innerhalb des Schlauches in eine Richtung gezielt gefördert wird. Die Schläuche in der Dispensier-Kassette müssen mit einer bestimmten Spannung auf dem Rotor der Peristaltikpumpe gespannt sein, um die Verformung der Schläuche durch den Rotor überhaupt zu ermöglichen.

Sowohl das Einstellen der Schlauchspannung für jeden einzelnen Schlauch als auch das schrittweise Anbringen der Kartuschen führen zu einem erheblichen Aufwand bei der Herstellung wie auch bei der Installation der Dispensierkassette an einer Peristaltikpumpe. Dieser Zeitaufwand überträgt sich direkt auf die Produktionszeit, was sich wiederum in einem ineffizienten Produktionsprozess widerspiegelt.

In US D 826 284 S ist ein Design einer Dispensier-Kassette zur Verwendung in einer Peristaltikpumpe gezeigt. Die Figuren in der Design-Anmeldung zeigen zwei Kartuschen, welche über ein U-Förmiges Mittelstück verbunden sind. Im Innern des U-Förmigen Mittelstücks sind Schläuche zwischen den Kartuschen angebracht. Die Schläuche münden in je ein Sammelrohr, welche in beiden Kartuschen in deren Längsrichtung angeordnet sind. Aus der Produktbeschreibung des Anmelders geht hervor, dass die Vorrichtung eine nahezu pulsationsfreie Dosierung ermöglicht.

### AUFGABE

Es ist deshalb eine Aufgabe der vorliegenden Erfindung, eine alternative Dispensierkassette zur Verfügung zu stellen, welche eine einfachere Bedienung als diejenigen aus dem Stand der Technik aufweist.

### BESCHREIBUNG

Die Aufgabe wird gelöst mit einer Dispensier-Kassette zum Anbringen an einer Peristaltikpumpe mit einer ersten Kartusche, einer zweiten Kartusche und Schläuchen, welche von der Dispensier-Kassette derart aufgenommen werden, dass beide Enden der Schläuche je in einer Kartusche angebracht sind. Die zwei Kartuschen sind über ein elastisches Mittelstück miteinander verbunden. Die erste Kartusche weist Aussparungen auf, um Dispensierspitze aufzunehmen, wobei die Düsen am Ende des Schlauchs angeordnet sind.

Aufgrund des elastischen Mittelstückes mit den beiden Kartuschen an dessen Enden handelt es sich bei der Dispensier-Kassette abgesehen von den Schläuchen um ein einstückiges Bauteil. Das elastische Mittelstück ermöglicht eine stetige Verbindung zwischen den Kartuschen unabhängig davon, ob ein Schlauch zwischen den Kartuschen angeordnet ist. Durch die Elastizität des Mittelstückes können sich die Kartuschen gegeneinander bis zu einem gewissen Grad frei bewegen. Dieser Grad der freien Bewegung ist durch die Ausführung des Mittelstücks bestimmt. Das Mittelstück kann sowohl translatorische als auch rotatorische Bewegungen der Kartuschen gegeneinander erlauben. Des Weiteren stellt das Mittelstück einen Schutz für die Schläuche dar. Somit unterliegen die Schläuche keiner zusätzlichen Beanspruchung nebst derjenigen beim Erfüllen ihrer Aufgabe für das Fördern des Mediums durch die Peristaltik. Die Elastizität des Mittelstückes ermöglicht weiterhin einen flexiblen Einsatz, so dass die Dispensier-Kassette an Peristaltikpumpen mit unterschiedlichen Gestalten und Formen angebracht werden kann.

Die Dispensier-Kassette ist vorgesehen, an einer Peristaltikpumpe angebracht zu werden. Die Dispensier-Kassette muss die Peristaltikpumpe bei deren Aufgabe ein flüssiges Medium zu fördern und dieses gezielt abzugeben unterstützen. Das Abgeben des flüssigen Mediums geschieht idealerweise über Düsen. Diese ermöglichen eine genauere Dosierung des Mediums. Das Medium sollte ohne zusätzlichen Widerstand vom Schlauch in die Düse gefördert werden. In der ersten Kartusche sind unter anderem deshalb Aussparungen angebracht. Die Aussparungen definieren eine Stelle, an welcher die Düse anzubringen ist, um einen möglichst widerstandslosen Übergang des Mediums vom Schlauch in die Düse zu erzielen.

Im Rahmen dieser Anmeldung ist unter dem Begriff Kartusche eine Vorrichtung für die Aufnahme von einem oder mehreren Schläuchen zu verstehen. Dabei kann der Schlauch durch die Kartusche durchgeführt sein oder aber auch innerhalb der Kartusche enden. Die Kartusche dient unter anderem einer genauen Platzzuordnung der Schläuche in einer Peristaltikpumpe, so dass sie eine Bewegung der Schläuche untereinander innerhalb der Kartusche verhindert. Des Weiteren ermöglicht die Kartusche synchrone peristaltische Bewegungen der Schläuche durch den Rotor.

In einer bevorzugten Ausführungsform ist das elastische Mittelstück U-förmig ausgebildet und an seinen beiden Enden ist je eine Kartusche angebracht. Die U-Form des Mittelstücks bietet eine gute Voraussetzung für dessen Elastizität. Mit einer Auswahl eines geeigneten Materials kann bereits alleine aufgrund der U-Form ein elastisches Verhalten des Mittelstücks erzielt werden. Das auf diese Weise hergestellte Mittelstück weist eine einfache Herstellbarkeit auf, wodurch die Herstellungskosten ebenfalls tief bleiben.

Die Kartuschen an den beiden Enden des Mittelstückes kommen vorteilhafterweise parallel zueinander zu liegen. Das elastische Mittelstück ist somit zwischen zwei Längsseiten der Kartuschen angeordnet. In einer solchen Ausführung kann die Breite der Dispensier-Kassette durch die Länge der Kartuschen gebildet sein.

In einer weiteren bevorzugten Ausführungsform sind die Schläuche lösbar an den beiden Kartuschen angebracht. Die Schläuche sind in einer Peristaltikpumpe für das Fördern eines Mediums zuständig. Aufgrund der höheren Belastung der Schläuche weisen diese eine kürzere Lebensdauer als die Kartuschen mit dem elastischen Mittelstück der Dispensier-Kassette auf. Somit ermöglicht eine Trennung der Schläuche von der restlichen Dispensier-Kassette weiterhin Gebrauch zu machen, indem neue Schläuche an den beiden Kartuschen angebracht werden. Das Auswechseln von Schläuchen muss auch nicht unbedingt am Ende der Lebensdauer der Schläuche stattfinden. Falls zum Beispiel die Anforderung an die Geometrie der Schläuche sich ändert, kann durch einen Wechsel der Schläuche darauf schnellstmöglich reagiert werden. Vorzugsweise weisen die Schläuche eine formschlüssige Verbindung mit den Kartuschen auf. Dafür können Aussparungen an den Kartuschen vorgesehen sein. Eine formschlüssige Verbindung ermöglicht das Erstellen und das Lösen einer Verbindung innerhalb einer kurzen Zeit.

Vorzugsweise weist die erste Kartusche die gleiche Anzahl an Aussparungen für die Aufnahme von Schläuchen und Düsen auf. Bevorzugt ist stromabwärts jedes Schlauches eine Düse angeordnet. Damit wird das zu dispensierende Medium separat in den einzelnen Schläuchen transportiert und über die jeweilige Düse nach aussen gefördert. Die Dispensier-Kassette ist vorgesehen, mit Schläuchen, welche eine möglichst kleine Abweichung ihrer Durchmesser untereinander aufweisen, betrieben zu werden. Aufgrund der in etwa identischen Durchmesser der in der Dispensier-Kassette eingesetzten Schläuchen kann auf ein Sammelrohr- oder -becken nach den Schläuchen verzichtet werden. Das Sammelrohr nach den Schläuchen dient dazu die Diskrepanz in der Fördermenge zwischen den Schläuchen und die entstehenden Pulsationen zu minimieren. Da aufgrund der beinahe identischen Durchmesser der Schläuche in der hier gezeigten Ausführung die Fördermenge zwischen den Schläuchen ebenfalls beinahe identisch ist, kann das Medium aus jedem einzelnen Schlauch über eine stromabwärts davon angeordnete Düse nach aussen gefördert werden. Dies führt zu einer einfacheren Bedienung und zu einem grösseren Freiraum bei der Verwendung der Dispensier-Kassette. Bevorzugt spannen die Schläuche in der Dispensier-Kassette einen U-förmigen Bogen. In einer Peristaltikpumpe findet das Fördern des Mediums durch Verformung der Schläuche durch den Rotor der Peristaltikpumpe statt. Um die Verformung der Schläuche durch den Rotor gewährleisten zu können, müssen die Schläuche über den Rotor gespannt sein. In einer solchen Anordnung kommen die Kartuschen der Dispensier-Kassette auf beinahe gegenüberliegenden Längsseiten des Rotors zu liegen. Das Spannen der Schläuche ermöglicht nebst dem Fördern des flüssigen Mediums in den Schläuchen durch Peristaltik auch einen definitiven Verschluss der Schläuche. Da die Schläuche in der Dispensier-Kassette auf einem Rotor zu liegen kommen, bietet sich die Anordnung der Schläuche in einem U-förmigen Bogen an. Mit dem Winkel des Bogens kann die Belastung auf die Schläuche gegebenenfalls eingestellt werden. Die Rotoren in den Peristaltikpumpen können sich in ihrem Aufbau unterscheiden. Deshalb ist es auch vorstellbar, dass die unterschiedlichen Rotoren bei der Auslegung der Dispensier-Kassette berücksichtigt werden und unter anderem der Winkel der Schläuche dem jeweiligen Rotor entsprechend bestimmt wird.

Vorzugsweise liegen die Schläuche innerhalb des U-förmigen Mittelstücks. Durch die U-Form des Mittelstücks ist ein innerer Bereich des Mittelstücks definiert, welcher durch das Mittelstück selbst und die imaginäre Verbindungslinie zwischen den an beiden Enden des Mittelstücks angebrachten Kartuschen gebildet ist. Die Schläuche sind nun vorzugsweise vorgesehen, in diesem inneren Bereich des Mittelstücks zu liegen. Dadurch bildet das elastische Mittelstück einen Schutz der Schläuche, so dass diese lediglich durch den inneren Bereich des U-förmigen Mittelstücks erreicht werden können. Der Zugriff auf die Schläuche von ausserhalb des Mittelstücks ist somit verhindert. Dies ermöglicht es die Dispensier-Kassette zu ergreifen, ohne Gefahr zu laufen, mit den Schläuchen in Berührung zu kommen und diese dadurch zu beschädigen. Zugleich können die Schläuche in einer Konstellation wie oben beschrieben nicht in unbeabsichtigter Weise auf Druck belastet werden. Auf Zug können die Schläuche auch nur soweit belastet werden, wie es das Mittelstück zulässt.

In einer weiteren bevorzugten Ausführungsform ist die zweite Kartusche vorgesehen, einen oder mehrere Zufuhr-Schläuche aufzunehmen, wobei der Zufuhr-Schlauch das zu transportierende Medium zur Dispensier-Kassette fördert. Da die Zufuhr-Schläuche nicht der gleichen Beanspruchung wie die Schläuche in der Dispensier-Kassette ausgesetzt sind, können sie eine geringe Belastbarkeit aufweisen.

Die Anzahl der Zufuhr-Schläuche ist vorteilhafterweise gleich der Anzahl der Schläuche zwischen den Kartuschen in der Dispensier-Kassette, so dass der Inhalt eines Zufuhr-Schlauchs innerhalb der zweiten Kartusche einem Schlauch übergeben werden kann. Die gleiche Anzahl an Zufuhr-Schläuchen erleichtert das zu dispensierende Medium in gleichen Mengen zur Dispensier-Kassette zu transportieren. Damit muss das Medium nicht nochmals zusätzlich in die einzelnen Schläuche verzweigt werden, wodurch der Strömungswiderstand für das Medium minimiert wird.

In einer anderen bevorzugten Ausführungsform kann die Dispensier-Kassette einen Verteiler vor der zweiten Kartusche aufweisen. Die Aufgabe des Verteilers ist die Aufnahme des zu dispensierenden Mediums und dessen Aufteilung und Transport durch die Kanäle des Verteilers zu den Schläuchen in der zweiten Kartusche. Der Verteiler kann dabei eine oder mehrere Aufnahmeöffnungen aufweisen. Die Anzahl der Kanäle ist vorteilhafter gleich der Anzahl an Schläuchen in der Dispensier-Kassette, so dass jedem Schlauch ein Zufuhr-Kanal zugeordnet werden kann.

In einer weiteren bevorzugten Ausführungsform weist die Dispensier-Kassette eine Breite von bis zu 15 cm auf und kann einhändig bedient werden. Die feste Verbindung zwischen den Kartuschen durch das elastische Mittelstück erzeugt einen einzigen zusammengehörenden Bauteil. Durch Einschränken der Breite der Dispensier-Kassette auf 15 cm wird aufgrund des einstückigen Aufbaus die einhändige Bedienung der Dispensier-Kassette ermöglicht. Somit kann die Dispensier-Kassette schneller an der Peristaltikpumpe angebracht und auch wieder gelöst werden. Die Breite der Dispensier-Kassette ist die Ausbreitung in senkrechter Richtung zur Verbindungslinie zwischen den Kartuschen.

Vorteilhafterweise umfasst die Dispensier-Kassette ein RFID-Modul, welches mit der Peristaltikpumpe kommunizieren kann. Ein RFID-Modul kann relevante Daten für den Betrieb der Peristaltikpumpe enthalten. Eine dieser Daten ist zum Beispiel der Pumpfaktor der Dispensier-Kassette. Dies ist ein Faktor, welcher von den Schläuchen der Dispensier-Kassette abhängt und die Fördermenge des Mediums bei bestimmten Randbedingungen festlegt.
Die Dispensier-Kassette kann im RFID-Modul betriebsrelevante Daten aufweisen. Diese Daten werden beim Anbringen der Dispensier-Kassette an eine Peristaltikpumpe an diese mittels des RFID-Moduls übermittelt. Somit kann die Peristaltikpumpe das Einstellen der Parameter zur Förderung des Mediums aufgrund der Daten aus dem RFID-Modul der Dispensier-Kassette vornehmen. Ohne das RFID-Modul müssten die Parameter der Peristaltikpumpe manuell eingestellt werden.

Die Dispensier-Kassette kann bevorzugt bis zu 32 Schläuche aufnehmen. Das heisst, dass an jeder der beiden Kartuschen 32 Schläuche angebracht sind. Die Schläuche können sowohl alle in einer Ebene liegen als auch zueinander versetzt angeordnet sein. Bei einer versetzten Anordnung können die Schläuche im Querschnitt alle zusammen eine runde oder eine rechteckige Form aufweisen.

Vorzugsweise weisen die Enden der Schläuche Verdickungen auf, welche in Aussparungen der Kartuschen aufgenommen werden. Die Aussparungen können bei der Herstellung oder mit einer Nachbearbeitung an den Kartuschen angebracht werden. Die Form der Aussparungen muss jeweils an die Form der Verdickungen der Schläuche angepasst werden. Die Aussparungen ermöglichen das Anbringen der Schläuche an den Kartuschen, ohne ein weiteres Hilfsmittel verwenden zu müssen. Die Verdickungen an den Schläuchen können auf unterschiedliche Art und Weise hergestellt werden. Vorstellbar ist, dass die Schläuche mit den Verdickungen mittels einer Gussform gespritzt werden. Des Weiteren ist es auch möglich, dass die Schläuche zum Beispiel in einem Extrusionsverfahren ohne Verdickungen hergestellt werden und die Verdickungen anschliessend auf den Schlauch geklebt werden.
Die Verdickungen sind vorgesehen, an den Enden der Schläuche platziert zu sein. Jedoch können die Verdickungen auch vom Ende des Schlauches einen Abstand aufweisen. Dieser Abstand steht im Vergleich zur Länge des Schlauches in einem Verhältnis von maximal 1:10.

Vorteilhafterweise sind die Schläuche in der Dispensier-Kassette parallel zu einander angeordnet. Die parallele Zuordnung der Schläuche untereinander verhindert, dass sich die Schläuche kreuzen können. Die Ordnung zwischen den Schläuchen bleibt stets aufrechterhalten und sorgt damit, dass die Schläuche keiner zusätzlichen Belastung ausgesetzt werden.
Des Weiteren sorgt die stets parallele Anordnung der Schläuche dafür, dass jeder Schlauch von der ersten Kartusche zur zweiten Kartusche die gleiche Distanz zurücklegt. Dies ist insofern von grosser Bedeutung, da der Widerstand auf das flüssige Medium von der Länge des Schlauches abhängt und deshalb unterschiedliche Schlauch-Längen untereinander zu unterschiedlichen Fördermengen führen würde.

In einer weiteren bevorzugten Ausführungsform sind die Schläuche an den beiden Kartuschen fest angebracht. Somit weisen die Schläuche eine feste Verbindung mit beiden Kartuschen auf. Die Dispensier-Kassette ist somit einstückig. Ein Vorteil einer solchen einstückigen Dispensier-Kassette ist die einfache Ersetzbarkeit einer bestimmten Dispensier-Kassette durch eine andere Dispensier-Kassette. Da die Schläuche ein Teil der Dispensier-Kassette sind, kann der Dispensier-Kassette als solche, ohne eine weitere detaillierte Betrachtung der Schläuche durchzuführen, eine entsprechende Fördermenge zugewiesen werden. Dies kann im Betrieb den Vorteil mit sich bringen, dass der Einsatz und Austausch einer Dispensier-Kassette schneller vorgenommen werden kann. Ein grosser Vorteil ergibt sich auch für die Herstellung. Durch den einstückigen Aufbau der Dispensier-Kassette mit den Schläuchen eignet sich diese besser für die automatische Produktion. Damit kann eine höhere Stückzahl innerhalb der gleichen Zeit hergestellt werden, wodurch sich der Herstellungskosten pro Stück reduziert.

Vorzugsweise gehen die Schläuche eine stoffschlüssige Verbindung mit den beiden Kartuschen ein. Die Art der stoffschlüssigen Verbindung hängt stark von den verwendeten Materialien ab. Beim Einsatz von unterschiedlichen Kunststoffen kann der Stoffschluss das Ergebnis einer Herstellung durch Spritzguss oder auch das Ergebnis eines Schmelzvorgangs bereits hergestellter Halbzeuge sein. Vorstellbar ist, dass die Schläuche auf die Dispensier-Kassette aufgespritzt werden, wobei die Verbindung der Schläuche zur Dispensier-Kassette über die beiden Kartuschen hergestellt wird. Damit kann während der Herstellung der Kartuschen und des elastischen Mittelstücks die Verbindung der Schläuche zu den Kartuschen gleichzeitig erzeugt werden. Die Produktionszeit wird dadurch gekürzt und eine eindeutige Zuordnung der Fördermenge zur Dispensier-Kassette kann aufgrund der festen Verbindung zwischen den Schläuchen und den Kartuschen vorgenommen werden.

Beide Kartuschen weisen vorteilhafterweise auf den zur Längsrichtung senkrecht stehenden Seiten vorzugsweise mindestens einen in Längsrichtung abstehenden Vorsatz auf. Der Vorsatz kann als Rastelement dienen. Die Längsrichtung ist durch jene Richtung definiert, in welcher die Schläuche nebeneinander angeordnet sind. Der abstehende Vorsatz bzw. das Rastelement dient zur Herstellung der Verbindung der Dispensier-Kassette an eine Peristaltikpumpe. In einer Ausführung mit einer möglichst einfachen Bedienung, kann über die Vorsätze bzw. Rastelemente eine Formschlussverbindung zwischen der Dispensier-Kassette und der Peristaltikpumpe erstellt werden. Dies ermöglicht die Dispensier-Kassette mit nur einer Hand auf eine einfache Weise an der Peristaltikpumpe zu installieren.

### KURZBESCHREIBUNG DER FIGUREN

Es zeigen in nicht massstabsgetreuer, schematischer Darstellung:
- Figur 1:: eine dreidimensionale Ansicht einer dreiteiligen Dispensier-Kassette aus dem Stand der Technik;
- Figur 2:: eine dreidimensionale Ansicht einer erfindungsmässigen Dispensier-Kassette mit 8 Schläuchen;
- Figur 3:: eine Seitenansicht einer Dispensier-Kassette, welche zur Installation an einer Peristaltikpumpe bereit ist;
- Figur 4:: eine dreidimensionale Ansicht einer erfindungsmässigen Dispensier-Kassette mit einem zweiteiligen Mittelstück; und
- Figur 5:: eine Explosionsdarstellung einer Dispensier-Kassette mit einem zweiteiligen Mittelstück.

### DETAILIERTE BESCHREIBUNG DER FIGUREN

Im Folgenden stehen gleiche Bezugsziffern für gleiche oder funktionsgleiche Elemente (in unterschiedlichen Figuren). Ein zusätzlicher Apostroph kann zur Unterscheidung gleichartiger bzw. funktionsgleicher oder funktionsähnlicher Elemente in einer weiteren Ausführung dienen.

In Figur 1 ist eine Dispensier-Kassette 11 aus dem Stand der Technik abgebildet. Die Dispensier-Kassette 11 umfasst drei Kartuschen 13, 15, 17, welche in der gezeigten Darstellung lediglich über einen Schlauch 19 miteinander in Verbindung stehen. Die Kartuschen 13,15,17 sind quaderförmig ausgebildet. Die Breite 23 verläuft parallel zur Axialrichtung der Schläuche 19. Die Breite 23 und der Abstand zwischen den Kartuschen ist derart gewählt, dass die Schläuche 19 zwischen zwei Kartuschen eine Umlenkung von 180° vornehmen können ohne den Durchfluss durch die Schläuche 19 zu beeinträchtigen. Die Länge 21 der Kartuschen wird durch die Anzahl an aufzunehmenden Schläuchen 19 bestimmt. Da die Schläuche 19 parallel in Richtung der Breite 23 angeordnet sind, verhält sich die Länge 21 der Kartusche linear zur Anzahl an von ihr aufnehmbaren Schläuchen 19. Die Höhe der Kartusche wird ebenfalls durch die Schläuche bestimmt. Sie misst abgesehen von der Wandstärke der Kartusche leicht mehr als der Aussendurchmesser der Schläuche. Für die Platzierung der Schläuche sind feste Sitze 25 an der ersten und ihr gegenüberliegenden zweiten Schmalseite vorgesehen. Die Schmalseiten werden jeweils durch eine Längs- und eine Höhenkante aufgespannt. Jede Kartusche 13, 15, 17 umfasst zwei Teile, einen Behälter 27 und einen Deckel. In Figur 1 sind lediglich die Behälter 27 der drei Kartuschen abgebildet. Der Deckel kann eine flächengespiegelte Vorrichtung des Behälters 27 sein, wobei die Flachseite, welche durch die Längskante 21 und die Breite 23 des Behälters aufgespannt ist, die Spiegelfläche bildet. Durch den zweiteiligen Aufbau der Kartusche wird es möglich, die Schläuche in die Kartusche einzulegen. Aus diesem Grund ist es wichtig, dass der Behälter 27 eine Öffnung 29 aufweist, welche einen freien Zugang zu allen darin platzierten Schläuchen bietet. Diese Öffnung 29 wird nach Einlegen der Schläuche durch den Deckel der Kartusche geschlossen. In der zweiten und dritten Kartusche 15, 17 ist ein Spannelement 31 für jeden Schlauch vorgesehen, wobei in Figur 1 nur ein Schlauch 19 mit den jeweiligen Spannelementen 31 gezeigt ist. Das Spannelement 31 kann über eine Stellschraube bedient werden. Durch das Spannelement 31 kann die auf den Schlauch 19 wirkende Spannung variiert werden. Dies ist erforderlich, um durch die Änderung der Spannung am Schlauch 19 dessen Innendurchmesser anzupassen, da die Schläuche 19 aufgrund der hohen Herstellungstoleranzen untereinander unterschiedliche Innendurchmesser aufweisen können.

In Figur 2 ist eine erfindungsmässige Dispensier-Kassette 11 gezeigt. Im Gegensatz zur Dispensier-Kassette 11 aus dem Stand der Technik ist eine erste Kartusche 13 über ein elastisches Mittelstück 33 mit einer zweiten Kartusche 15 verbunden. Auf eine dritte Kartusche 17, wie sie aus dem Stand der Technik bekannt ist, wird verzichtet. Die Aufgabe der dritten Kartusche 17 wird in der erfindungsmässigen Dispensier-Kassette 11 von der zweiten Kartusche 15 übernommen. Die erste 13 als auch die zweite 15 Kartusche sind Quaderförmige Bauteile, deren Längskante 21 ein Vielfaches der anderen zwei Kanten ist. Die Kartuschen 13, 15 sind derart angeordnet, dass ihre Längskanten 21 parallel zueinander verlaufen. Beide Kartuschen 13, 15 weisen an ihrer Oberseite Öffnungen 35 auf, durch welche die Schläuche geführt sind. Die erste Kartusche 13 weist gegenüber der zweiten Kartusche 15 eine grössere Breite auf. Die zweite Kartusche 15 nimmt nicht nur die Schläuche 19 zur ersten Kartusche 13 sondern auch die Zufuhr-Schläuche 37 auf. Deren Anzahl ist gleich gross, wie diejenige der Schläuche 19 zwischen der ersten 13 und zweiten Kartusche 15. Die Zufuhr-Schläuche 37 sind senkrecht zur Oberseite der Kartusche angeordnet. Sie zweigen alle von einem Hauptschlauch 39 ab, welcher parallel zu den Längskanten 21 der Kartuschen verläuft. Die Aufgabe des Hauptschlauches 39 besteht darin, das zu dispensierende Medium von einem Behälter (nicht gezeigt) zu den Zufuhr-Schläuchen 37 zu fördern. Vorstellbar ist auch, dass die Zufuhr-Schläuche 37 das zu dispensierende Medium direkt vom Behälter beziehen und auf die Verwendung eines Hauptschlauchs verzichtet wird. Ein Vorteil einer solchen Ausführung ohne Hauptschlauch wäre unter anderem die Möglichkeit, die Zufuhrschläuche einzeln ansteuern bzw. betreiben zu können.

Das Mittelstück 33 ist als ein U-förmiges Bauteil gebildet, welches an beiden Enden an je einer Kartusche 13, 15 angebracht ist. Die Breite des Mittelstückes ist in etwa gleich gross wie die Längskante 21 der Kartusche. Die Breite des Mittelstückes wird auf der oberen Seite der Kartuschen in deren Mitte längsseitig angebracht. Das Mittelstück 33 weist im nicht gebogenen Zustand eine rechteckige Grundform auf, wobei die Länge und Breite ein Vielfaches der Höhe betragen.

Das Mittelstück 33 umfasst ein elastisches Material, welches sich derart verformen kann, dass sich die Enden des Mittelstücks 33 mit den daran angeordneten Kartuschen zueinander bewegen können. In seiner Mitte weist das Mittelstück 33 einen Kamm 41 auf.

Dieser verläuft auf der dem Innenbereich des U-förmigen Bauteils abgeneigten Seite des Mittelstücks parallel zur Richtung der Längskanten 21 der Kartuschen. Der Kamm 41 weist eine Dicke auf, welche gleich gross ist wie die Dicke des elastischen Mittelstücks 33 in dessen anderen Bereichen. Die Höhe des Kamms 41 ist so gewählt, dass ein Mensch mit seinen Fingern den Kamm 41 greifen kann.

Die erste Kartusche 13 weist auf Ihrer nach aussen hin ausgerichteten Längsseite Aussparungen 43 auf. Die Aussparungen 43 befinden sich nahe am Rand aber klar oberhalb der Längskante. Die Aussparungen 43 werden in der Längsrichtung der Kartusche 15 wiederholt von Streben unterbrochen. In den Aussparungen kommen Düsenspitzen 45 zum Vorschein. Jede Düsenspitze 45 in der ersten Kartusche 13 weist eine dichte Verbindung mit einem Schlauch 19 auf, so dass die Düsenspitze 45 das Medium aus dem Schlauch 19 im Wesentlichen verlustfrei aufnehmen und fördern kann.

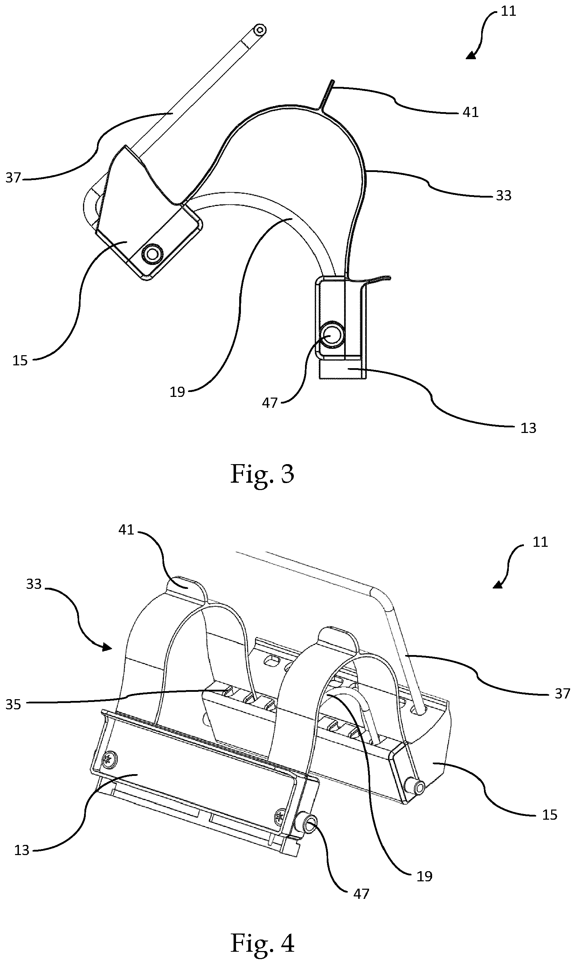

In Figur 3 ist die Dispensier-Kassette 11 von der Seite gezeigt. Die Schläuche 19, welche zwischen der ersten 13 und der zweiten Kartusche 15 angeordnet sind, weisen eine kürzere Länge als das Mittelstück 33 auf. Dies führt dazu, dass die Schläuche 19 sich stets innerhalb des vom U-förmigen Bauteil aufgespannten Innenbereich befinden. Die Kanten beider Kartuschen 13, 15 weisen Fasen auf. Auf der in Figur 3 abgebildeten Seite beider Kartuschen ist jeweils ein zylinderförmiger Vorsatz 47 angebracht. Die Zylinderachse des Vorsatzes 47 ist parallel zur Längskante 21 der Kartusche angeordnet. Der Vorsatz 47 umfasst eine Aussparung in der Mitte, so dass der Vorsatz 47 durch eine Zylinderwand gebildet ist. Der Vorsatz 47 der ersten Kartusche 13 ist grösser als derjenige der zweiten Kartusche 15. Der Vorsatz 47 kann als Rastelement zur Herstellung der Verbindung mit einer Peristaltikpumpe dienen.

Im Gegensatz zu den Dispensier-Kassetten 11 aus dem Stand der Technik ist es möglich, dass die in Figur 3 gezeigte Dispensier-Kassette 11 kein Spannelement für das Einstellen der auf einen Schlauch wirkenden Spannung verfügt. Die bevorzugte Kombination einer in Figur 2 und Figur 3 gezeigten Dispensier-Kassette ist jene mit Schläuchen, welche eine derart geringe Streuung ihres Durchmessers aufweisen, dass an allen Schläuchen die gleiche Spannung angelegt werden kann.

In Figur 4 ist eine Dispensier-Kassette 11 mit einer unterschiedlichen Ausführung des elastischen Mittelstücks 33 gezeigt. Dieses weist einen zweiteiligen Aufbau auf. Die Kartuschen 13, 15 und die Schläuche 19 weisen den gleichen Aufbau beziehungsweise die gleiche Zuordnung auf. Gegenüber dem in den Figuren 2 und 3 gezeigten Mittelstück 33 deckt das Mittelstück 33 aus Figur 4 nicht die ganze Länge der Kartuschen 13, 15 ab, sondern jeweils nur einen begrenzten Bereich an den beiden Enden der Kartuschen 13,15. Die Form des Mittestücks 33 aus Figur 4 ist bis auf die Grösse der Breite die gleiche wie diejenige aus den Figuren 2 und 3. Die Breite des Mittelstücks ist etwa um ein fünf bis sechsfaches reduziert, so dass wie in der Figur 4 gezeigten Ausführung das Mittelstück aus zwei Teilen bestehen kann. Vorstellbar ist, dass das Mittelstück aus einer grösseren Anzahl an Teilen besteht. Dafür müssten diese Teile eine derart kleine Breite aufweisen, dass alle auf einer Seite der Kartusche angebracht werden könnten. Auch in einer mehrteiligen Ausführung weist das elastische Mittelstück in seiner Gesamtheit vorzugsweise eine Breite auf, die mindestens 10%, noch bevorzugter mindestens 15%, der Länge der Kartuschen ist.

In Figur 4 ist lediglich eine Schlauchverbindung vom Zufuhrschlauch bis zur Düse gezeigt. In der Anwendung der Dispensier-Kassette 11 ist davon auszugehen, dass die restlichen Schläuche auch an der Dispensier-Kassette 11 angebracht sind. Bei einem mehrteiligen Aufbau des elastischen Mittelstücks 33 kann die Überdeckung aller Schläuche 19 durch das elastische Mittelstück 33 nicht unbedingt gewährleistet werden. Bei einem zweiteiligen Aufbau des elastischen Mittelstücks 33 wie in Figur 4 können die Kartuschen 13,15 mit einem geringeren Widerstand gegeneinander verdreht werden als bei einem einteiligen Aufbau des elastischen Mittelstücks 33. Dies wird durch das Anordnen der Teile des elastischen Mittelstücks 33 an den Enden der Kartuschen 13,15, welche miteinander auch nicht verbunden sind, ermöglicht.

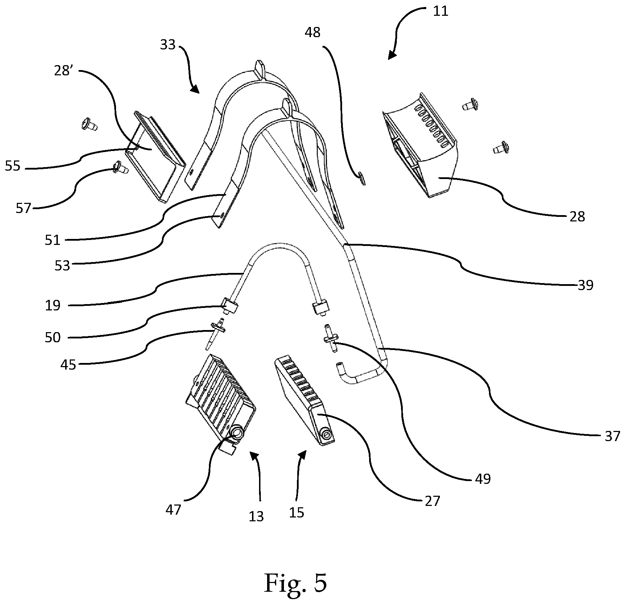

In Figur 5 ist eine Explosionsdarstellung einer Dispensier-Kassette 11 mit deren wichtigsten Bestandteilen gezeigt. Die Kartuschen 13,15 weisen einen zwei-stückigen Aufbau auf wie bereits diejenigen aus dem Stand der Technik. Der Behälter 27 der Kartusche wird durch dasjenige Stück gebildet, welches für die Aufnahme der Schläuche zuständig ist. Das zweite Stück bildet den Deckel 28, welcher derart auf dem Behälter 27 angeordnet ist, dass die Schläuche 19 dadurch in ihren Positionen fixiert werden. Die Schlauchverbindung umfasst einen Hauptschlauch 39, einen Zufuhr-Schlauch 37 und einen zwischen den beiden Kartuschen angeordneten Schlauch 19. In der Explosionsdarstellung ist nur eine Schlauchverbindung gezeigt. In der Anwendung können jedoch mehrere Schlauchverbindungen verwendet werden, wobei die Anzahl des Hauptschlauches 39 sich nicht verändert und eins bleibt. Zwischen dem Zufuhr-Schlauch 37 und dem Schlauch 19 zwischen den Kartuschen 13, 15 ist ein Verbindungsstück 49 angeordnet. Dieses umfasst einen kurzen Rohrabschnitt, dessen Aussenwände an beiden Enden einen konischen Verlauf aufweisen. Das Verbindungsstück 49 ist vorgesehen, mit beiden Enden in je einen der zu verbindenden Schläuche eingeführt zu werden. Durch Überziehen des Schlauches über das Verbindungsstück 49 stellt sich aufgrund des konischen Verlaufs der Aussenwand des Verbindungsstücks und der Elastizität des Schlauches eine Reibschluss-Verbindung ein. Am anderen Ende des Schlauches 19 kann eine Düse 45 angebracht werden. Diese weist wie das Verbindungsstück ebenfalls eine konische Aussenwand auf, damit sie auch in den Schlauch 19 eingeschoben werden kann. An dem vom Schlauch gegenüberliegenden Ende der Düse ist eine Verengung sowohl des Aussen- als auch des Innendurchmessers vorgesehen. Damit wird die Querschnittsfläche reduziert, was beim Dispensieren das Nachtropfen der Flüssigkeit hemmt. Der Schlauch 19 weist an beiden Enden Verdickungen 50 in Radialrichtung auf. Die Verdickungen 50 haben eine quaderförmige Form und einen etwa zwei bis dreimal so grossen Querschnitt wie der Schlauch 19. In den Kartuschen 13, 15 sind Aussparungen vorgesehen, welche in Figur 5 nicht ersichtlich sind und welche die Verdickungen der Schläuche aufnehmen. Dadurch entsteht eine formschlüssige Verbindung zwischen dem Schlauch 19 und der Kartusche 13,15, womit der Schlauch 19 innerhalb der jeweiligen Kartusche 13, 15 fixiert ist.
In der zweiten Kartusche 15 ist ein RFID-Tag 48 vorgesehen. Das RFID-Tag kann betriebsrelevante Informationen der Dispensier-Kassette 11 aufweisen, wie zum Beispiel den Pumpfaktor. Die Information im RFID-Tag kann kabellos auf einen benachbarten Bauteil transferiert werden. Dieser Bauteil kann unter anderem die Peristaltikpumpe sein, in welcher die Dispensier-Kassette angeordnet wird.
Das elastische Mittelstück 33 besteht wie bereits in Figur 4 auch in Figur 5 aus zwei Teilen. Diese weisen an beiden Enden jeweils eine Verlängerung 51 auf, welche im verbauten Zustand nicht ersichtlich sind. An diesen Verlängerungen 51 ist je ein Loch 53 angebracht. In den Deckeln 28, 28' der beiden Kartuschen 13,15 sind auch jeweils Durchbohrungen 55 vorgesehen, so dass im zusammengesetzten Zustand der Dispensier-Kassette 11 die Durchbohrung im Deckel der Kartusche auf dem Loch 53 im elastischen Mittelstück zu liegen kommt. Durch die beiden Löcher wird eine Schraube 57 geführt, welche im Behälter 27 der Kartusche fest angebracht wird und somit für den Zusammenhalt aller Bauteile sorgt.

### BEZUGSZEICHENLISTE:

- 11: Dispensier-Kassette
- 13: Erste Kartusche
- 15: Zweite Kartusche
- 17: Dritte Kartusche
- 19: Schlauch
- 21: Längskante der Kartusche
- 23: Breite der Kartusche
- 25: Sitze für Platzierung der Schläuche
- 27: Behälter der Kartusche
- 28. 28': Deckel der Kartusche
- 29: Öffnung des Behälters der Kartusche
- 31: Spannelement
- 33: Elastisches Mittelstück
- 35: Öffnungen auf Oberseite der Kartuschen
- 37: Zufuhr-Schläuche
- 39: Hauptschlauch
- 41: Kamm des Mittelstücks
- 43: seitliche Aussparungen in der ersten Kartusche
- 45: Düsenspitze
- 47: zylinderförmiger Vorsatz / Rastelement
- 48: RFID-Tag
- 49: Verbindungsstück
- 50: Verdickungen am Schlauch
- 51: Verlängerung des elastischen Mittelstücks
- 53: Loch im elastischen Mittelstück
- 55: Durchbohrung im Deckel der Kartusche
- 57: Schraube

## Patentansprüche

1. Dispensier-Kassette (11) zum Anbringen an einer Peristaltikpumpe mit
einer ersten Kartusche (13),
einer zweiten Kartusche (15)
und Schläuchen (19), welche von der Dispensier-Kassette (11) derart aufgenommen werden, dass beide Enden der Schläuche (19) je in einer Kartusche (13, 15) angebracht sind,
wobei die zwei Kartuschen (13, 15) über ein elastisches Mittelstück (33) miteinander verbunden sind,
**dadurch gekennzeichnet,**
**dass** die erste Kartusche (13) Aussparungen aufweist um Düsen aufzunehmen, wobei die Düsen am Ende des Schlauchs angeordnet sind.

2. Dispensier-Kassette (11) nach Anspruch 1, **dadurch gekennzeichnet, dass** das elastische Mittelstück (33) U-förmig ausgebildet ist und an seinen beiden Enden je eine Kartusche (13, 15) angebracht ist.

3. Dispensier-Kassette (11) nach Anspruch 1 oder 2, **dadurch gekennzeichnet, dass** die Schläuche (19) lösbar an den beiden Kartuschen (13, 15) angebracht sind.

4. Dispensier-Kassette (11) nach einem der Ansprüche 1 bis 3, **dadurch gekennzeichnet, dass** die erste Kartusche die gleiche Anzahl an Aussparungen für die Aufnahme von Schläuchen und Düsen aufweist.

5. Dispensier-Kassette (11) nach einem der Ansprüche 1 bis 4, **dadurch gekennzeichnet, dass** die Schläuche (19) in der Dispensier-Kassette einen U-förmigen Bogen spannen.

6. Dispensier-Kassette (11) nach einem der Ansprüche 1 bis 5, **dadurch gekennzeichnet, dass** die Schläuche (19) innerhalb des U-förmigen Mittelstücks (33) liegen.

7. Dispensier-Kassette (11) nach einem der Ansprüche 1 bis 6, **dadurch gekennzeichnet, dass** die erste Kartusche (13) vorgesehen ist, einen oder mehrere Zufuhr-Schläuche (37) aufzunehmen, wobei der Zufuhr-Schlauch (37) das zu transportierende Medium zur Dispensier-Kassette (11) fördert.

8. Dispensier-Kassette (11) nach einem der Ansprüche 1 bis 7, **dadurch gekennzeichnet**, die Anzahl der Zufuhr-Schläuche (37) gleich der Anzahl der Schläuche (19) zwischen den Kartuschen (13, 15) in der Dispensier-Kassette (11) ist, so dass der Inhalt eines Zufuhr-Schlauchs (37) innerhalb der zweiten Kartusche (15) einem Schlauch (19) übergeben wird.

9. Dispensier-Kassette (11) nach einem der Ansprüche 1 bis 8, **dadurch gekennzeichnet, dass** die Dispensier-Kassette (11) eine Breite von bis zu 15 cm aufweist und einhändig bedient werden kann.

10. Dispensier-Kassette (11) nach einem der Ansprüche 1 bis 9, **dadurch gekennzeichnet, dass** die Dispensier-Kassette (11) ein RFID-Modul umfasst, welches mit der Peristaltikpumpe kommunizieren kann.

11. Dispensier-Kassette (11) nach einem der Ansprüche 1 bis 10, **dadurch gekennzeichnet, dass** die Dispensier-Kassette (11) bis zu 32 Schläuche (19) aufnehmen kann.

12. Dispensier-Kassette (11) nach einem der Ansprüche 1 bis 11, **dadurch gekennzeichnet, dass** die Enden der Schläuche (19) Verdickungen (50) aufweisen, welche in Aussparungen der Kartuschen (13, 15) aufgenommen werden.

13. Dispensier-Kassette (11) nach einem der Ansprüche 1 bis 12, **dadurch gekennzeichnet, dass** die Schläuche (19) in der Dispensier-Kassette (11) parallel zu einander angeordnet sind.

14. Dispensier-Kassette (11) nach einem der Ansprüche 1 bis 13, **dadurch gekennzeichnet, dass** die Schläuche (19) an den beiden Kartuschen (13, 15) fest angebracht sind.

15. Dispensier-Kassette (11) nach Anspruch 14, **dadurch gekennzeichnet, dass** die Schläuche (19) eine stoffschlüssige Verbindung mit den beiden Kartuschen (13, 15) eingehen.
